# EUROPEAN PATENT APPLICATION

(11) **EP 1 538 141 A1**
(43) Date of publication of application: **08.06.2005**
(21) Application number: 03795291.8
(22) Date of filing: 05.09.2003
(51) Int. Cl.: C07C 67/317, C07C 69/757, C07C 51/377, C07C 62/02, C07C 62/16

(54) **PROCESS FOR PRODUCTION OF 2-(HYDROXYMETHYL)CYCLO- PROPANECARBOXYLIC ACIDS**

(30) Priority: 10.09.2002 JP 2002263700
(71) Applicant: Sumitomo Chemical Company, Limited, Tokyo 104-8260 (JP)
(72) Inventor: MINAMIDA, Ryo, Katano-shi, Osaka 576-0036 (JP); ITAGAKI, Makoto, Katano-shi, Osaka 576-0066 (JP)
(74) Representative: Henkel, Feiler & Hänzel
(86) International application number: PCT/JP2003/011330
(87) International publication number: WO 2004/024667

(57) **Abstract**

There is provided a production method of a compound of formula (2): 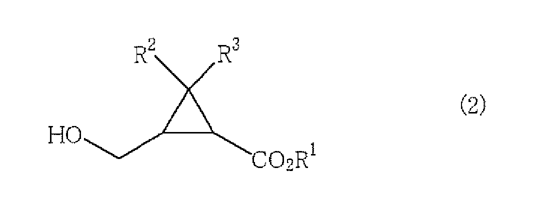 wherein R¹, R² and R³ are as defined below,
which comprises reacting
a 2-(hydroxymethyl)cyclopropanecarboxylic compound of formula (1): 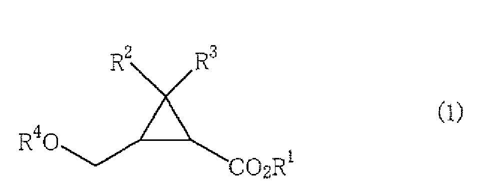 wherein and R¹ represent a hydrogen, a linear, branched or cyclic alkyl group, a substituted or unsubstituted aryl group,
R² and R³ are the same or different and represent a hydrogen atom or a methyl group, and
R⁴ represents C1-2 alkyl group substituted with at lest one group selected from the group consisting of a substituted aryl group and an unsubstituted aryl group,
with a hydrogen-donor in the presence of a catalyst selected from the group consisting of ruthenium catalyst, cobalt catalyst, rhodium catalyst, nickel catalyst, palladium catalyst and a platinum catalyst.

## Description

### Technical Field

The present invention relates to a process for production of a 2-(hydroxymethyl)cyclopropanecarboxylic compound, which is an useful intermediate compound for the production of chrysanthemic acid derivatives.

### Background Art

There is disclosed in Scheme 2 described in Tetrahedron: Asymmetry, 1995, 6, page 648 a production method of methyl 2-(hydroxymethyl)cyclopropanecarboxylate from a compound that was produced through multi-steps starting from glutamic acid as the starting material. There is also disclosed in Scheme 1 described in Tetrahedron, 2001, 57, page 6086 a method by selectively hydrolyzing, in the presence of a base, a diester compound resulting from acetylation of the hydroxyl group of ethyl 2-(hydroxymethyl)-3,3-dimethylcyclopropanecarboxylate. However, as the production method of 2-(hydroxymethyl)cyclopropanecarboxylic derivative, the former method has a problem in that it has multi-steps and the synthesis of the starting material was complicated and the flatter method has problems in that the selectivity was not always satisfactory. Hence, an advantageous industrial production method has been desired.

### Disclosure of the Invention

According to the method of the present invention, the (2-hydroxymethyl)cyclopropanecarboxylic compound of formula (2) below can be produced advantageously in industrial production.

That is, the present invention provides a production method of a compound of formula (2): 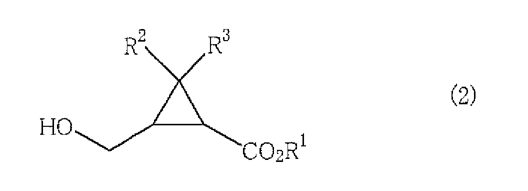 wherein R¹, R² and R³ are as defined below,
which comprises reacting
a 2-(hydroxymethyl)cyclopropanecarboxylic compound of formula (1): 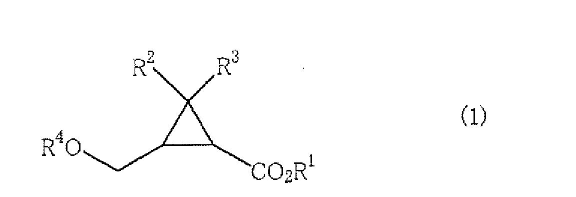 wherein and R¹ represent a hydrogen, a linear, branched or cyclic alkyl group, a substituted or unsubstituted aryl group,
R² and R³ are the same or different and represent a hydrogen atom or a methyl group, and
R⁴ represents C1-2 alkyl group substituted with at lest one group selected from the group consisting of a substituted aryl group and an unsubstituted aryl group,
with a hydrogen-donor in the presence of a catalyst selected from the group consisting of ruthenium catalyst, cobalt catalyst, rhodium catalyst, nickel catalyst, palladium catalyst and a platinum catalyst.

### Best Mode for Carrying Out the Invention

In formula (1) or (2), examples of the linear, branched or cyclic alkyl group represented by R¹ include, for example, a C1-15 linear, branched or cyclic alkyl group such as methyl, ethyl, n-propyl, isopropyl, n-butyl, isobutyl, sec-butyl, tert-butyl, n-pentyl, n-hexyl, cyclohexyl, n-heptyl, n-octyl, n-nonyl, n-decyl, menthyl, undecyl, dodecyl, tridecyl, tetradecyl, pentadecyl, or the like.

Examples of the unsubstituted aryl group represented by R¹ include, for example, phenyl, naphthyl or the like, and examples of the substituted aryl include, for example, aryl, as described above for unsubstituted aryl, substituted with at least one group selected from the group consisting of
the alkyl group described above (preferably, C1-4 alkyl such as methyl, ethyl, n-propyl, isopropyl, n-butyl, isobutyl, sec-butyl, or tert-butyl),
a fluorine, an alkoxy group such as methoxy, ethoxy or the like,
an aryl group such as phenyl, naphthyl or the like,
an aryloxy group such as phenoxy or the like, and
an alkoxycarbonyl group such as methoxycarbonyl, ethoxycarbonyl or the like.

Specific examples thereof include, for example, 4-methylphenyl group, 2,6-di(tert-butyl)-4-methylphenyl group, a 4-phenoxyphenyl group and the like.

R¹ is preferably linear, branched or cyclic alkyl, and more preferably C1-4 alkyl such as methyl, ethyl, n-propyl, isopropyl, n-butyl, isobutyl, sec-butyl, or tert-butyl.

Examples of the unsubstituted aryl contained in the C1-2 alkyl (preferably methyl) substituted with at least one group selected from the group consisting of substituted aryl and unsubstituted aryl include, for example, phenyl, and naphthyl (preferably phenyl).

Examples of the substituted aryl include, for example, an aryl group substituted with at least one group selected from the group consisting of alkyl (e.g. C 1-4 alkyl such as methyl, ethyl, propyl, butyl, or the like), alkoxy(e.g. Cl-2 alkoxy such as methoxy, ethoxy or the like), and phenyl.

Specific examples thereof include, for example, benzyldiphenylmethyl, trityl, phenethyl (preferably 1-phenethyl), naphthylmethyl, 4-methoxybenzyl, 2,4-dimethoxybenzyl, 4-phenylbenzyl, (4-methoxyphenyl)diphenylmethyl, di(4-methoxyphenyl)phenylmethyl, tri(4-mehoxyphenyl)methyl and the like. Preferred is benzyl.

The cyclopropanecarboxylic compound (1) have two isomers, which are a cis-isomer having the group shown by -CO₂R¹ and the group shown by -CH₂OH on the same side with respect to the cyclopropane ring plane and a trans-isomer having the groups on the opposite side, and any one of the cis-isomer and the trans-isomer or a mixture thereof may be used in the present invention. In addition, the cyclopropanecarboxylate compound (1) has four optical isomers due to the presence of the two asymmetric carbon atoms and any one optical isomer thereof or a mixture of two or more of them may be used.

The compound of formula (1) can be produced in a similar manner known in the art (e.g. Tetrahedron, 2001, 57, 6083-6088 etc.) which comprises reacting a corresponding olefin compound with a diazoacetic ester compound in the presence of a metal catalyst.

Examples of the compound of formula (1) include, for example, 2-(benzyloxymethyl)cyclopropanecarboxylic acid, 2-[(4-methoxybenzyloxy)methyl]cyclopropanecarboxylic acid, 2-[2,4-(dimethoxybenzyloxy)methyl]cyclopropanecarboxylic acid, 2-[(4-phenylbenzyloxy)methyl]cyclopropanecarboxylic acid, 2-[(diphenylmethoxy)methyl]cyclopropanecarboxylic acid, 2-(trityloxymethyl)cyclopropanecarboxylic acid, 2-[[(4-methoxyphenyl)diphenylmethoxy]methyl]cyclopropanecarboxylic acid, 2-[[di(4-methoxyphenyl)phenylmethoxy]methyl]cyclopropanecarboxylic acid, 2-[[tri(4-methoxyphenyl)methoxy]methyl]cyclopropanecarboxylic acid, methyl 2-(benzyloxymethyl)cyclopropanecarboxylate, methyl 2-[(4-methoxybenzyloxy)methyl]cyclopropanecarboxylate, methyl 2-[2,4-(dimethoxybenzyloxy)methyl]cyclopropanecarboxylate, methyl 2-[(4-phenylbenzyloxy)methyl]cyclopropanecarboxylate, methyl 2-[(diphenylmethoxy)methyl]cyclopropanecarboxylate, methyl 2-(trityloxymethyl)cyclopropanecarboxylate, methyl 2-[[(4-methoxyphenyl)diphenylmethoxy]methyl]cyclopropanecarboxylate, methyl 2-[[di(4-methoxyphenyl)phenylmethoxy]methyl]cyclopropanecarboxylate, methyl 2-[[tri(4-methoxyphenyl)methoxy]methyl]cyclopropanecarboxylate, ethyl 2-(benzyloxymethyl)cyclopropanecarboxylate, ethyl 2-[(4-methoxybenzyloxy)methyl]cyclopropanecarboxylate, ethyl 2-[2,4-(dimethoxybenzyloxy)methyl]cyclopropanecarboxylate, ethyl 2-[(4-phenylbenzyloxy)methyl]cyclopropanecarboxylate, ethyl 2-[(diphenylmethoxy)methyl]cyclopropanecarboxylate, ethyl 2-(trityloxymethyl)cyclopropanecarboxylate, ethyl 2-[[(4-methoxyphenyl)diphenylmethoxy]methyl]cyclopropanecarboxylate, ethyl 2-[[di(4-methoxyphenyl)phenylmethoxy]methyl]cyclopropanecarboxylate, ethyl 2-[[tri(4-methoxyphenyl)methoxy]methyl]cyclopropanecarboxylate, tert-butyl 2-(benzyloxymethyl)cyclopropanecarboxylate, tert-butyl 2-[(4-methoxybenzyloxy)methyl]cyclopropanecarboxylate, tert-butyl 2-[2,4-(dimethoxybenzyloxy)methyl]cyclopropanecarboxylate, tert-butyl 2-[(4-phenylbenzyloxy)methyl]cyclopropanecarboxylate, tert-butyl 2-[(diphenylmethoxy)methyl]cyclopropanecarboxylate, tert-butyl 2-(trityloxymethyl)cyclopropanecarboxylate, tert-butyl 2-[[(4-methoxyphenyl)diphenylmethoxy]methyl]cyclopropanecarboxylate, tert-butyl 2-[[di(4-methoxyphenyl)phenylmethoxy]methyl]cyclopropanecarboxylate, tert-butyl 2-[[tri(4-methoxyphenyl)methoxy]methyl]cyclapropanecarboxylate, cyclohexyl 2-(benzyloxymethyl)cyclopropanecarboxylate, cyclohexyl 2-[(4-methoxybenzyloxy)methyl]cyclopropanecarboxylate, cyclohexyl 2-[2,4-(dimethoxybenzyloxy)methyl]cyclopropanecarboxylate, cyclohexyl 2- [(4-phenylbenzyloxy)methyl]cyclopropanecarboxylate, cyclohexyl 2-[(diphenylmethoxy)methyl]cyclopropanecarboxylate, cyclohexyl 2-(trityloxymethyl)cyclopropanecarboxylate, cyclohexyl 2-[[(4-methoxyphenyl)diphenylmethoxy]methyl]-cyclopropanecarboxylate, cyclohexyl 2-[di(4-methoxyphenyl)phenylmethoxy]methyl]cyclopropanecarboxylate, cyclohexyl 2-[tri(4-methoxyphenyl)methoxy]methyl]cyclopropanecarboxylate, menthyl 2-(benzyloxymethyl)cyclopropanecarboxylate, menthyl 2-[(4-methoxybenzyloxy)methyl]cyclopropanecarboxylate, menthyl 2-[(2,4-dimethoxybenzyloxy)methyl]cyclopropanecarboxylate, menthyl 2-[(4-phenylbenzyloxy)methyl]cyclopropanecarboxylate, menthyl 2-[(diphenylmethoxy)methyl]cyclopropanecarboxylate, menthyl 2-(trityloxymethyl)cyclopropanecarboxylate, menthyl 2-[[(4-methoxyphenyl)diphenylmethoxy]methyl]cyclopropanecarboxylate, menthyl 2-[[di(4-methoxyphenyl)phenylmethoxy]methyl]cyclopropanecarboxylate, menthyl 2-[[tri(4-methoxyphenyl)methoxy]methyl]cyclopropanecarboxylate, 2,6-di(tert-butyl)-4-methylphenyl 2-(benzyloxymethyl)cyclopropanecarboxylicacid, 2,6-di(tert-butyl)-4-methylphenyl 2-[(4-methoxy benzyloxy)methyl]cyclopropanecarboxylate, 2,6-di(tert-butyl)-4-methylphenyl 2-[2,4-(dimethoxybenzyloxy)methyl]-cyclopropanecarboxylate, 2,6-di(tert-butyl)-4-methylphenyl 2-[(4-phenylbenzyloxy)-methyl]cyclopropanecarboxylate, 2,6-di(tert-butyl)-4-methylphenyl 2-[(diphenylmethoxy)-methyl]cyclopropanecarboxylate, 2,6-di(tert-butyl)-4-methylphenyl 2-(trityloxymethyl)cyclopropanecarboxylate, 2,6-di(tert-butyl)-4-methylphenyl 2-[[(4-methoxyphenyl)-diphenylmethoxy]methyl]cyclopropanecarboxylate, 2,6-di(tert-butyl)-4-methylphenyl 2-[[di(4-methoxyphenyl)-phenylmethoxy]methyl]cyclopropanecarboxylate, 2,6-di(tert-butyl)-4-methylphenyl 2-[[tri(4-methoxyphenyl)-methoxy]methyl]cyclopropanecarboxylate, 2-(benzyloxymethyl)-3-methylcyclopropanecarboxylic acid, 2-[(4-methoxybenzyloxy)methyl]-3-methylcyclopropanecarboxylic acid, 2-[2,4-(dimethoxybenzyloxy)methyl-3-methylcyclopropanecarboxylic acid, 2-[(4-phenylbenzyloxy)methyl-3-methylcycloproponecarboxylic acid, 2-[(diphenylmethoxy)methyl-3-methylcyclopropanecarboxylic acid, 2-(trityloxymethyl)-3-methylcyclopropanecarboxylic acid, 2-[[(4-methoxyphenyl)diphenylmethoxy]methyl-3-methylcyclopropanecarboxylic acid, 2-[[di(4-methoxyphenyl)phenylmethoxy]methyl-3-methylcyclopropanecarboxylic acid, 2-[[tri(4-methoxyphenyl)methoxy]methyl]-3-methylcyclopropanecarboxylic acid, methyl 2-(benzyloxymethyl)-3-methylcyclopropanecarboxylate, methyl 2-[(4-methoxybenzyloxy)methyl]-3-methylcyclopropanecarboxylate, methyl 2-[(2,4-dimethoxybenzyloxy)methyl]-3-methylcyclopropanecarboxylate, methyl 2-[(4-phenylbenzyloxy)methyl]-3-methyl-cyclopropanecarboxylate, methyl 2-[(diphenylmethoxy)methyl-]-3-methylcyclopropanecarboxylate, methyl 2-(trityloxymethyl)-3-methylcyclopropanecarboxylate, methyl 2-[[(4-methoxyphenyl)diphenylmethoxy]methyl]-3-methylcyclopropanecarboxylate, methyl 2-[[di(4-methoxyphenyl)phenylmethoxy]methyl-3-methylcyclopropanecarboxylate, methyl 2-[[tri(4-methoxyphenyl)methoxy]methyl-3-methylcyclopropanecarboxylate, ethyl 2-(benzyloxymethyl)-3-methylcyclopropanecarboxylate, ethyl 2-[(4-methoxybenzyloxy)methyl-3-methylcyclopropanecarboxylate, ethyl 2-(2,4-dimethoxybenzyloxy)methyl-3-methylcyclopropanecarboxylate, ethyl 2-[(4-phenylbenzyloxy)methyl-3-methylcyclopropanecarboxylate, ethyl 2-[(diphenylmethoxy)methyl]-3-methylcyclopropanecarboxylate, ethyl 2-(trityloxymethyl)-3-methylcyclopropanecarboxylate, ethyl [[(4-methoxyphenyl)diphenylmethoxy]methyl]-3-methylcyclopropanecarboxylate, ethyl 2-[[di(4-methoxyphenyl)phenylmethoxy]methyl-3-methylcyclopropanecarboxylate, 2-[[tri(4-methoxyphenyl)methoxy]methyl-3-methylcyclopropanecarboxylate, tert-butyl 2-(benzyloxymethyl)-3-methylcyclopropanecarboxylate, tert-butyl 2-[(4-methoxybenzyloxy)methyl-3-methylcyclopropanecarboxylate, tert-butyl 2-[(2,4-dimethoxybenzyloxy)methyl]-3- methylcyclopropanecarboxylate, tert-butyl 2-[(4-phenylbenzyloxy)methyl-3-methylcyclopropanecarboxylate, tert-butyl 2-(diphenylmethoxy)methyl-3-methylcyclopropanecarboxylate, tert-butyl 2-[[di(4-nitrophenyl)methoxy]methyl-3-methylcyclopropanecarboxylate, tert-butyl 2-(trityloxymethyl)-3-methylcyclopropanecarboxylate, tert-butyl 2-[[(4-methoxyphenyl)diphenylmethoxy]methyl-3- methylcyclopropanecarboxylate, tert-butyl 2-[[di(4-methoxyphenyl)phenylmethoxy]methyl-3-methylcyclopropanecarboxylate, tert-butyl 2-[[tri(4-methoxyphenyl)methoxy]methyl-3- methylcyclopropanecarboxylate, cyclohexyl 2-(benzyloxymethyl)-3-methylcyclopropanecarboxylate, cyclohexyl 2-[(4-methoxybenzyloxy)methyl-3-methylcyclopropanecarboxylate, cyclohexyl 2-[(2,4-dimethoxybenzyloxy)methyl]-3-methylcyclopropanecarboxylate, cyclohexyl 2-[(4-phenylbenzyloxy)methyl]-3-methylcyclopropanecarboxylate, cyclohexyl 2-[(diphenylmethoxy)]methyl-3-methylcyclopropanecarboxylate, cyclohexyl 2-(trityloxymethyl)-3-methylcyclopropanecarboxylate, cyclohexyl 2-[(4-methoxyphenyl)diphenylmethoxy]methyl-3-methylcyclopropanecarboxylate, cyclohexyl 2-[[di(4-methoxyphenyl)phenylmethoxy]methyl-3-methylcyclopropanecarboxylate, cyclohexyl 2-[[tri(4-methoxyphenyl)methoxy]]methyl-3-methylcyclopropanecarboxylate, menthyl 2-(benzyloxymethyl)-3-methylcyclopropanecarboxylate, menthyl 2-[(4-methoxybenzyloxy)methyl]-3-methylcyclopropanecarboxylate, menthyl 2-[(2,4-dimethoxybenzyloxy)methyl]-3-methylcyclopropanecarboxylate, menthyl 2-[(4-phenylbenzyloxy)methyl]-3-methylcyclopropanecarboxylate, menthyl 2-[(diphenylmethoxy)methyl]-3-methylcyclopropanecarboxylate, menthyl 2-(trityloxymethyl)-3-methylcyclopropanecarboxylate, menthyl 2-[[(4-methoxyphenyl)diphenylmethoxy]methyl]-3- methylcyclopropanecarboxylate, menthyl 2-[di(4-methoxyphenyl)phenylmethoxy]methyl-3- methylcyclopropanecarboxylate, menthyl 2-[[tri(4-methoxyphenyl)methoxy]methyl-3- methylcyclopropanecarboxylate, 2,6-di(tert-butyl)-4-methylphenyl 2-(benzyloxymethyl)-3-methylcyclopropanecarboxylate, 2,6-di(tert-butyl)-4-methylphenyl 2-[(4-methoxybenzyloxy)methyl]-3-methylcyclopropanecarboxylate, 2,6-di(tert-butyl)-4-methylphenyl 2-[(2,4-dimethoxybenzyloxy) methyl]-3-methylcyclopropanecarboxylate, 2,6-di(tert-butyl)-4-methylphenyl 2-[(4-phenylbenzyloxy- methyl)-3-methylcyclopropanecarboxylate, 2,6-di(tert-butyl)-4-methylphenyl 2-[(diphenylmethoxy)- methyl]-3-methylcyclopropanecarboxylate, 2,6-di(tert-butyl-4-methylphenyl 2-(trityloxymethyl)-3-methylcyclopropanecarboxylate, 2,6-di(tert-butyl)-4-methylphenyl 2-[(4-methoxyphenyl)-diphenylmethoxy]methyl-3-methylcyclopropanecarboxylate, 2,6-di(tert-butyl)-4-methylphenyl 2-[di(4-methoxyphenyl)-phenylmethoxy] methyl-3-methylcyclopropanecarboxylate, 2,6-di(tert-butyl)-4-methylphenyl 2-[tri(4-methoxyphenyl)-methoxy]methyl]-3-methylcyclopropanecarboxylate, 2-(benzyloxymethyl)-3,3-dimethylcyclopropanecarboxylic acid, 2-[(4-methoxybenzyloxy)methyl]-3,3-dimethylcyclopropanecarboxylic acid, 2-[(2,4-dimethoxybenzyloxy)methyl]-3,3-dimethylcyclopropanecarboxylic acid, 2-[(4-phenylbenzyloxy)methyl]-3,3-dimethylcyclopropanecarboxylic acid, 2-[(diphenylmethoxy)methyl]-3,3-dimethylcyclopropanecarboxylic acid, 2-(trityloxymethyl)-3,3-dimethylcyclopropanecarboxylic acid, 2-[[(4-methoxyphenyl)diphenylmethoxy]-3,3-dimethylcyclopropanecarboxylic acid, 2-[di(4-methoxyphenyl)phenylmethoxy]methyl-3,3-dimethylcyclopropanecarboxylic acid, 2-[[tri(4-methoxyphenyl)methoxy]-3,3- dimethylcyclopropanecarboxylic acid, methyl 2-(benzyloxymethyl)-3,3-dimethylcyclopropanecarboxylate, methyl 2-[(4-methoxybenzyloxy)methyl]-3,3-dimethylcyclopropanecarboxylate, methyl 2-[(2,4-dimethoxybenzyloxy)methyl]-3,3-dimethylcyclopropanecarboxylate, methyl 2-[(4-phenylbenzyloxy)methyl]-3,3-dimethylcyclopropanecarboxylate, methyl 2-[(diphenylmethoxy)methyl]-3,3-dimethylcyclopropanecarboxylate, methyl 2-(trityloxymethyl)-3,3-dimethylcyclopropanecarboxylate, methyl 2-[[(4-methoxyphenyl)diphenylmethoxy]methyl-3,3-dimethylcyclopropanecarboxylate, methyl 2-[[di(4-methoxyphenyl)phenylmethoxy]methyl-3,3-dimethylcyclopropanecarboxylate, methyl 2-[[tri(4-methoxyphenyl]methoxy]methyl-3,3-dimethylcyclopropanecarboxylate, ethyl 2-(benzyloxymethyl)-3,3-dimethylcyclopropanecarboxylate, ethyl 2-(4-methoxybenzyloxy)methyl-3,3-dimethylcyclopropanecarboxylate, ethyl 2-[(2,4-dimethoxybenzyloxy)methyl]-3,3-dimethylcyclopropanecarboxylate, ethyl 2-[(4-phenylbenzyloxy)methyl]-3,3-dimethylcyclopropanecarboxylate, ethyl 2-[(diphenylmethoxy)methyl]-3,3-dimethylcyclopropanecarboxylate, ethyl 2-(trityloxymethyl)-3,3-dimethylcyclopropanecarboxylate, ethyl 2-[[(4-methoxyphenyl)diphenylmethoxy]methyl]-3,3-dimethylcyclopropanecarboxylate, ethyl 2-[[di(4-methoxyphenyl)phenylmethoxy]methyl-3,3-dimethyl cyclopropanecarboxylate, ethyl 2-[[tri(4-methoxyphenyl)methoxy]methyl-3,3-dimethylcyclopropanecarboxylate, tert-butyl 2-(benzyloxymethyl)-3,3-dimethylcyclopropanecarboxylate, tert-butyl 2-(4-methoxybenzyloxy)methyl-3,3-dimethylcyclopropanecarboxylate, tert-butyl 2-[(2,4-dimethoxybenzyloxy)methyl]-3,3- dimethylcyclopropanecarboxylate, tert-butyl 2-[(4-phenylbenzyloxy)methyl]-3,3-dimethylcyclopropanecarboxylate, tert-butyl 2-[(diphenylmethoxy)methyl]-3,3-dimethylcyclopropanecarboxylate, tert-butyl 2-(trityloxymethyl)-3,3-dimethylcyclopropanecarboxylate, tert-butyl 2-[[(4-methoxyphenyl)diphenylmethoxy]methyl]-3,3-dimethylcyclopropanecarboxylate, tert-butyl 2-[[di(4-methoxyphenyl)phenylmethoxy]methyl] -3,3-dimethylcyclopropanecarboxylate, tert-butyl 2-[[tri(4-methoxyphenyl)methoxy]methyl]-3,3-dimethylcyclopropanecarboxylate, cyclohexyl 2-(benzyloxymethyl)-3,3-dimethylcyclopropanecarboxylate, cyclohexyl 2-[(4-methoxybenzyloxy)methyl]-3,3-dimethylcyclo propanecarboxylate, cyclohexyl 2-[(2,4-dimethoxybenzyloxy)methyl]-3,3-dimethylcyclopropanecarboxylate, cyclohexyl 2-[(4-phenylbenzyloxy)methyl]-3,3-dimethylcyclopropanecarboxylate, cyclohexyl 2-[(diphenylmethoxy)methyl]-3,3-dimethylcyclopropanecarboxylate, cyclohexyl 2-(trityloxymethyl)-3,3-dimethylcyclopropanecarboxylate, cyclohexyl 2-[[(4-methoxyphenyl)diphenylmethoxy]methyl-3,3-dimethylcyclopropanecarboxylate, cyclohexyl 2-[[di(4-methoxyphenyl)phenylmethoxy]methyl-3,3-dimethylcyclopropanecarboxylate, cyclohexyl 2-[[tri(4-methoxyphenyl)methoxy]methyl]-3,3-dimethylcyclopropanecarboxylate, menthyl 2-(benzyloxymethyl)-3,3-dimethylcyclopropanecarboxylate, menthyl 2-[(4-methoxybenzyloxy)methyl]-3,3-dimethylcyclopropanecarboxylate, menthyl 2-[(2,4-dimethoxybenzyloxy)methyl]-3,3-dimethylcyclopropanecarboxylate, menthyl 2-(4-phenylbenzyloxy)methyl-3,3-dimethylcyclopropanecarboxylate, menthyl 2-[(diphenylmethoxy)methyl-3,3-dimethylcyclopropanecarboxylate, menthyl 2-(trityloxymethyl)-3,3-dimethylcyclopropanecarboxylate, menthyl 2-[[(4-methoxyphenyl)diphenylmethoxy]methyl]-3,3-dimethylcyclopropanecarboxylate, menthyl 2-[[di(4-methoxyphenyl)phenylmethoxy]methyl]-3,3-dimethylcyclopropanecarboxylate, menthyl 2-[[tri(4-methoxyphenyl)methoxy]methyl]-3,3-dimethylcyclopropanecarboxylate, 2,6-di(tert-butyl)-4-methylphenyl 2-(benzyloxymethyl)-3,3-dimethylcyclopropanecarboxylate, 2,6-di(tert-butyl)-4-methylphenyl 2-[(4-methoxybenzyloxy)methyl]-3,3-dimethylcyclopropanecarboxylate, 2,6-di(tert-butyl)-4-methylphenyl 2-[(2,4-dimethoxybenzyloxy)methyl]-3,3-dimethylcyclopropanecarboxylate, 2,6-di(tert-butyl)-4-methylphenyl 2-(4-phenylbenzyloxy)-methyl-3,3-dimethylcyclopropanecarboxylate, 2,6-di(tert-butyl)-4-methylphenyl 2-[(diphenylmethoxy)-methyl]-3,3-dimethylcyclopropanecarboxylate, 2,6-di(tert-butyl)-4-methylphenyl 2-[di(4-nitrophenyl)-methoxy]methyl]-3,3-dimethylcyclopropanecarboxylate, 2,6-di(tert-butyl)-4-methylphenyl 2-(trityloxymethyl)-3,3-dimethylcyclopropanecarboxylate, 2,6-di(tert-butyl)-4-methylphenyl 2-[(4-methoxyphenyl)-diphenylmethoxy]methyl]-3,3-dimethylcyclopropanecarboxylate, 2,6-di(tert-butyl)-4-methylphenyl 2-[di(4-methoxyphenyl)-phenylmethoxy]methyl]-3,3-dimethylcyclopropanecarboxylate, 2,6-di(tert-butyl)-4-methylphenyl 2-[[tri(4-methoxyphenyl)-methoxy]methyl]-3,3-dimethylcyclopropanecarboxylate.

The catalyst used in the present invention selected from the group consisting of ruthenium catalyst, cobalt catalyst, rhodium catalyst, nickel catalyst, palladium catalyst and a platinum catalyst mat be an non-homogeneous catalyst supported on a carrier such as activated carbon, alumina, or silica, or it may, for example, a homogeneous catalyst system comprising the metal or the metal compound and various coordinated ligands.

Examples of the ruthenium catalyst include, for example, ruthenium/alumina, ruthenium/carbon, chlorotris(triphenylphosphine) ruthenium and the like. Examples of the cobalt catalyst include, for example, raney-cobalt and the like. Examples of the rhodium catalyst include, for example,, rhodium/alumina, rhodium/carbon, chlorotris(triphenylphosphine) rhodium and the like. Examples of the nickel catalyst include, for example, Raney nickel, nickel/carbon, nickel/silica alumina, tetrakis(triphenylphosphine) nickel, bis(cyclooctadiene) nickel and the like. Examples of the palladium catalyst include, for example, palladium/carbon, palladium/alumina, tetrakis(triphenylphosphine) palladium, tris(dibenzylideneacetone) dipalladium, palladium hydroxide/carbon and the like. Examples of the platinum catalyst include, for example, platina/carbon, platina/alumina, tetrakis(triphenylphosphine) platina, bis(cyclooctadiene) platina and the like.

For the catalyst, a commercially available catalyst may be used as it is or such a catalyst as prepared according to a known process may be used. The amount of the catalyst that may be used is usually catalytic amount or may be in a range of 0.0001 to 1 mol per mol of the compound of formula (1).

As the hydrogen donor, hydrogen (H₂) is preferable, and as hydrogen donor, formic acid, ammonium formate, cyclohexene, cyclohexadiene, isopropanol are illustrated. Formic acid is preferred among the hydrogen donor except hydrogen.

As the catalyst, palladium catalyst is preferable, and palladium /carbon is preferable among them.

The nickel catalyst, particularly Raney nickel, are preferred catalyst among the catalyst other than the palladium catalyst, and is suitably used with hydrogen.

Hydrogen-donor may be added to the reaction system as it is, or may be used by dissolving or dispersing it in a solvent described below.

In addition, the hydrogen donor may be used alone or as a mixture thereof.

The amount of the hydrogen donor is usually 1 mol or more per mol of the compound of formula (1) and its upper limit is not particularly restricted.

The reaction is usually conducted by contacting the catalyst, the compound of formula (1) and the hydrogen donor, and the mixing order thereof is not limited. The reaction may be conducted at normal pressure or under pressurized condition.

The reaction may be carried out in absence of solvent, or it may be carried out in a solvent.

There is no limitation as to the solvent so long as the solvent does not inhibit the reaction, and examples of the solvent include, for example, alcohol solvent such as methanol, ethanol, isopropanol, or the like, an aromatic hydrocarbon solvent such as toluene, xylene, mesitylene or the like, a halogenated hydrocarbon solvent such as dichloromethane, chloroform, carbon tetrachloride, 1,2-dichloroethane or the like, ether solvent such as diethyl ether, diisoprpyl ether, methyl tert-butyl ether, or the like, an inert solvent such as water or the like, and mixtures of these solvents. The amount of the solvent is not particularly restricted.

The reaction temperature is usually about 0 °C to 100 °C.

The desired 2-(hydroxymethyl)cyclopropanecarboxylic compound of formula (2) can be isolated from the reaction mixture where the heterogeneous catalyst is employed, for example, by removing the catalyst by filtration and concentrating. Alternatively, the 2-(hydroxymethyl)cyclopropanecarboxylic compound of formula (2) can be isolated, for example, by distillating the reaction solution when homogeneous catalyst is employed. The isolated 2-(hydroxymethyl)cyclopropanecarboxylic compound of formula (2) may be further purified by distillation, column chromatography or the like. In production method of the present invention, 2- (hydroxymethyl) cyclopropanecarboxylic compound of formula (2) is obtained with retention of its cis-isomer/trans-isomer ratio when a mixture of cis-isomer/trans-isomer of the compound of formula (1) is used. When optically active compound is used as the compound of formula (1), optically active 2-(hydroxymethyl)cyclopropanecarboxylic compound of formula (2) is obtained with retention of the stereoisomerism.

Examples of the 2-(hydroxymethyl)cyclopropanecarboxylic compound of formula (2) thus produced include, for example, 2-(hydroxymethyl)cyclopropanecarboxylic acid, methyl 2-(hydroxymethyl)cyclopropanecarboxylate, ethyl 2-(hydroxymethyl)cyclopropanecarboxylate, tert-butyl 2-(hydroxymethyl)cyclopropanecarboxylate, cyclohexyl 2-(hydroxymethyl)cyclopropanecarboxylate, menthyl 2-(hydroxymethyl)cyclopropanecarboxylate, 2,6-di(tert-butyl)-4-methylphenyl 2-(hydroxymethyl)cyclopropanecarboxylate, 2-(hydroxymethyl)-3-methylcyclopropanecarboxylic acid, methyl 2-(hydroxymethyl)-3-methylcyclopropanecarboxylate, ethyl 2-(hydroxymethyl)-3-methylcyclopropanecarboxylate, tert-butyl 2-(hydroxymethyl)-3-methylcyclopropanecarboxylate, cyclohexyl 2-(hydroxymethyl)-3-methylcyclopropanecarboxylate, menthyl 2-(hydroxymethyl)-3-methylcyclopropanecarboxylate, methyl 2-(hydroxymethyl)-3-methylcyclopropanecarboxylate, 2,6-di(tert-butyl)-4-methylphenyl 2-(hydroxymethyl)-3-methylcyclopropanecarboxylate, 2-(hydroxymethyl)-3,3-dimethylcyclopropanecarboxylic acid, methyl 2-(hydroxymethyl)-3,3-dimethylcyclopropanecarboxylate, ethyl 2-(hydroxymethyl)-3,3-dimethylcyclopropanecarboxylate, tert-butyl 2-(hydroxymethyl)-3,3-dimethylcyclopropanecarboxylate, cyclohexyl 2-(hydroxymethyl)-3,3-dimethylcyclopropanecarboxylate, menthyl 2-(hydroxymethyl)-3,3-dimethylcyclopropanecarboxylate, methyl 2-(hydroxymethyl)-3,3-dimethylcyclopropanecarboxylate, 2,6-di(tert-butyl)-4-methylphenyl 2-(hydroxymethyl)-3,3-dimethylcyclopropanecarboxylate,

2-(Hydroxymethyl)cyclopropanecarboxylic compound of formula (2) thus produced can be further reacted with an oxidizer to produce corresponding 2-formylcyclorpopanecarboxylic compound. As the oxidizer, exemplified are oxidizers used for Swern oxidation (e.g. Tetrahedron, 2001, 57, 6038-6088) and pyridinium chlorochromate (e.g. Tetrahedron: Asymmetry, 1995, 6, 683-384), and a preferable oxidation method as disclosed in an International Application (PCT/JP03/08555).

### Example

The present invention is illustrated below in more detail with Examples, but it is not limited thereto. The analysis was conducted with gas chromatography, Internal standardization method

### Example 1

2.6 g of ethyl 2-(benzyloxymethyl)-3,3-dimethylcyclopropanecarboxylate, 100 mL of 4.4 wt% formic acid/methanol and 2.6 g of 10 wt% palladium/carbon were charged into a 200 mL of Schlenk tube and reacted under stirring at room temperature. Then the catalyst was removed by filtration with celite, and obtained filtrate was concentrated to give 1.7 g of ethyl 2-(benzyloxymethyl)-3,3-dimethylcyclopropanecarboxylate as a transparent oily substance.
Yield: 98 %

### Example 2

193 g of 23% aqueous sodium hydroxide solution were charged into a 1L four-neck flask under nitrogen atmosphere and heated to 110°C. 222 of a solution containing 48.9 % ethyl 2-(benzyloxymethyl)-3,3-dimethylcyclopropanecarboxylate were added dropwise to the aqueous solution over 3.5 hours and after the addition the resulting mixture was kept at 110 °C under heating and stirring for 6 hours. After cooled to room temperature, 333 ml of water and 111 ml of toluene were added thereto and settled to separate the crude aqueous solution of sodium 2-(hydroxymethyl)-3,3-dimethylcyclopropanecarboxylate. Phase separation was repeated thrice. 127 g of 35% hydrochloric acid and 111 ml of toluene were added to the aqueous solution and settled and separated to obtain a toluene solution of crude 2-(benzyloxymethyl)-3,3-dimethykcyclopropanecarboxylic acid. The phase separation was repeated thrice and obtained toluene solution was washed thrice with 111 ml of water, and concentrated to give 96.9 g of 2-(benzyloxymethyl)-3,3-dimethykcyclopropanecarboxylic acid.
Yield: 100%.

2.0 g of 2-(benzyloxymethyl)-3,3-dimethylcyclopropanecarboxylic acid, 26 mg of 10 wt% palladium/carbon and 10 mL of methanol were charged into a 20mL eggplant type flask, and hydrogen was charged thereto under normal pressure, and reacted under stirring for 2.5 hrs at 50 °C. Then the reaction solution was filtered and the filtrate was analyzed by high-performance liquid chromatography using Internal Standard Method, and it was found that 1.2 g of trans-2-(hydroxymethyl)-3,3-dimethylcyclopropanecarboxyic acid was contained.
Yield:
98%.

### Industrial Applicability

According to the method of the present invention, 2-(hydroxymethyl)cyclopropanecarboxylic compound, which is a useful intermediate for the synthesis of chrysanthemic acid derivative can be produced advantageously in industrial production.

## Claims

1. A production method of a compound of formula (2): 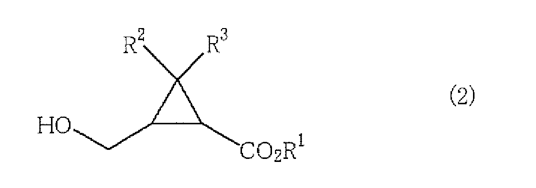 wherein R¹, R² and R³ are as defined below,
which comprises reacting
a 2-(hydroxymethyl)cyclopropanecarboxylic compound of formula (1): 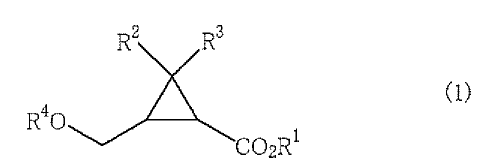 wherein and R¹ represent a hydrogen, a linear, branched or cyclic alkyl group, a substituted or unsubstituted aryl group,
R² and R³ are the same or different and represent a hydrogen atom or a methyl group, and
R⁴ represents C 1-2 alkyl group substituted with at lest one group selected from the group consisting of a substituted aryl group and an unsubstituted aryl group,
with a hydrogen-donor in the presence of a catalyst selected from the group consisting of ruthenium catalyst, cobalt catalyst, rhodium catalyst, nickel catalyst, palladium catalyst and a platinum catalyst.

2. A production method according to claim 1, wherein the catalyst is a nickel catalyst or palladium catalyst.

3. A production method according to claim 2, wherein the nickel catalyst is Raney-nickel.

4. A production method according to claim 2, wherein the palladium catalyst is palladium/carbon.

5. A production method according to any one of claims 1 to 4, wherein R¹ represent a C1-5 linear, branched or cyclic alkyl group, and
R⁴ represents an unsubstituted aryl group or an aryl group substituted with at least one group selected from the group consisting of alkyl, alkoxy, and phenyl.

6. A production method according to claim 1 or 4, wherein the hydrogen-donor is hydrogen or formic acid.

7. A production method according to any one of claims 1 to 4, wherein the hydrogen-donor is hydrogen.

8. A production method according to claim 1, which further comprises the step of reacting the resulting 2-(hydroxymethyl)cyclopropanecarboxylic compound of formula (2) with an oxidizing agent to produce corresponding 2-formylcyclopropanecarboxylate compound.
